# EUROPEAN PATENT SPECIFICATION

(11) **EP 1 583 547 B1**
(45) Date of publication and mention of the grant of the patent: **23.09.2009**
(21) Application number: 03779025.0
(22) Date of filing: 26.12.2003
(51) Int. Cl.: A61K 36/17, A61K 36/539, A61K 36/88, A61P 3/04

(54) **ANTI-OBESITY INGREDIENTS FROM MEDICINAL PLANTS AND THEIR COMPOSITION**
WIRKSTOFFE GEGEN ADIPOSITAS AUS MEDIZINISCHEN PFLANZEN UND DEREN ZUSAMMENSETZUNG
INGREDIENTS ANTI-OBESITE OBTENUS A PARTIR DE PLANTES MEDICINALES ET LEUR COMPOSITION

(30) Priority: 27.12.2002 KR 2002085058
(43) Date of publication of application: 12.10.2005
(73) Proprietor: Korea Institute of Oriental Medicine, Yuseong-gu Daejeon 305-811 (KR); SK Chemicals, Co., Ltd., Suwon-si, Gyeonggi-do 440-300 (KR)
(72) Inventor: YOON, Yoo Sik, Yeongdeungpo-gu Seoul (KR); KIM, Sun, Hyoung, 130-823 Seoul (KR); HAN, Chang-Kyun, 153-014 Seoul (KR); CHOI, Wonrack, Guro 3(sam)-dong-Guro-gu Seoul (KR); JEON, Hyo, Jin, 440-300 Kyungki-do (KR); IM, Guang-Jin, Ansan-si Gyeonggi-do (KR); KIM, Yong, Hyuk, 440-828 Kyungki-do (KR); KUM, Do, Seung, 135-092 Seoul (KR); KWAK, Wie-Jong, 137-044 Seoul (KR)
(74) Representative: Weber, Roland
(86) International application number: PCT/KR2003/002852
(87) International publication number: WO 2004/058284

(56) References cited:
- WO-A1-01/64229
- JP-A- 08 198 769
- KR-A- 98 067 315
- KR-A- 99 011 833
- KR-A- 2001 017 229
- KR-A- 2001 025 156
- KR-A- 2001 088 728
- KR-A- 2003 081 189
- US-A- 5 985 282
- US-B1- 6 475 530
- DATABASE WPI Week 200047 Derwent Publications Ltd., London, GB; AN 2000-515329 XP002436884 & CN 1 255 306 A (LI E) 7 June 2000 (2000-06-07)
- DATABASE WPI Week 199115 Derwent Publications Ltd., London, GB; AN 1991-102472 XP002436885 & CN 1 043 625 A (HANG WENJING) 11 July 1990 (1990-07-11)
- BOOZER C N ET AL: "Herbal ephedra/caffeine for weight loss: a 6-month randomized safety and efficacy trial." INTERNATIONAL JOURNAL OF OBESITY AND RELATED METABOLIC DISORDERS : JOURNAL OF THE INTERNATIONAL ASSOCIATION FOR THE STUDY OF OBESITY MAY 2002, vol. 26, no. 5, May 2002 (2002-05), pages 593-604, XP002436881 ISSN: 0307-0565
- CHO E.J. ET AL.: 'Purification and characterization of the inhibitory principle against pancreatic cholesterol esterase from Ephedra herba' J.KOREAN SOC. FOOD SCI. NUTR. vol. 28, no. 4, 1999, pages 816 - 821, XP008085408
- BOOZER C.N. ET AL.: 'Herbal ephedra/caffeine for weight loss: a 6-month randomized safety and efficacy trial' INT. J. OBESITY vol. 26, no. 5, May 2002, pages 593 - 604, XP002436881

## Description

### FIELD OF THE INVENTION

The present invention relates to a composition for preventing obesity comprising mixed herbal medicines, in particular, to a composition for inhibiting and preventing obesity, which consists of a mixed extract obtained from Ephedrae Herba, Scutellariae Radix and Typhae Pollen in defined weight ratios or the composition consists of the mixed extract described above together with at least one selected from the group consisting of Acorus gramineus Rhizome, Armeniacae Semen, Nelumbinus Folium and Polygalae Radix.

### DESCRIPTION OF THE RELATED ART

As living standards being upgraded, people tend to eat foods that contain high calories such as processed foods while being less actively indulged themselves in doing physical exercises, thus threatening their health conditions resulted from obesity. Obesity often gives bad images to others and also causes inconveniences in managing social activities. Furthermore, obesity has been known to increase the incidence of various diseases such as diabetes, arteriosclerosis, hypertension, heart disease, liver disease, cholelithiasis, gout, kidney disease, and also increases a risk at the time of a surgical operation. Therefore, obesity has drawn much attention of those who are middle aged or older because it is highly likely to develop various life-threatening diseases, whereas it is again raised as a most important issue for young women who would wish to keep their physical beauties at a desired level.

Recently, there has been an increasing interest on several medicinal ingredients, whose safety and efficacy have been approved through a number of generations, to use them for preventing and treating geriatric diseases such as obesity in addition to a suitable exercise. In fact, there have been various kinds of health foods introduced in commercial market which contain dietary fibers, ingredients for herbal medicines or several food materials allegedly being effective in treating obesity of people, however, most of the products have showed little efficacies. In addition, when people depend their regular diet largely on those dietary products while much reducing the amount of their regular food intake, it would be very difficult for them to manage their normal lives because they would probably suffer from many side effects such as lack , of nutrition. Meanwhile, if they stop taking those dietary products, their body will rapidly return to their original obese state much to their regret.

Accordingly, for overcoming the shortcomings of the dietary products described above, the present inventors have conducted extensive researches, and as a result, have found that the composition comprising a mixed extract of Ephedrae Herba, Scutellariae Radix and Typhae Pollen or the composition comprising the mixed extract described above together with at least one selected from the group consisting of Acorus gramineus Rhizome, Armeniacae Semen, Nelumbinus Folium and Polygalae Radix can prevent and remedy obesity.

Accordingly, it is an object of this invention to provide a composition consisting of herbal medicines for use to prevent and treat obesity and diseases associated with the same.

Further, it is another object of this invention to provide a therapeutic agent for treating obesity comprising the composition.

It is still another object of this invention to provide a health food comprising the composition.

### DETAILED DESCRIPTION OF THIS INVETNION

The present invention relates to a composition for preventing obesity, which comprises a mixed extract in defined weigth ratios, from Ephedrae Herba, Scutellariae Radix and Typhae Pollen or the composition comprising the mixed extract described above together with at least one selected from the group consisting of Acorus gramineus Rhizome, Armeniacae Semen, Nelumbinus Folium and Polygalae Radix.

In addition, the present invention relates to a therapeutic agent for use in treating obesity, and a health food comprising the composition.

The present invention will be described in more detail hereunder.

The present invention relates to a composition for preventing obesity, which comprises a mixed extract, in defined weigth ratios, obtained from Ephedrae Herba, Scutellariae Radix and Typhae Pollen or the composition comprising the mixed extract described above together with at least one selected from the group consisting of Acorus gramineus Rhizome, Armeniacae Semen, Nelumbinus Folium and Polygalae Radix.

The object of this invention described above to treat obesity can be accomplished by using the composition comprising as dry weight 30-60 weight parts of each of Ephedrae Herba, Scutellariae Radix and Typhae Pollen.

In addition, the object of this invention described above can be accomplished by using the composition comprising the mixed extract described above together with at least one selected from the group consisting of 30-60 weight parts of Acorus gramineus Rhizome, 30-60 weight parts of Armeniacae Semen, 15-45 weight parts of Nelumbinus Folium and 15-45 weight parts of Polygalae Radix.

The herbal medicines constituting the present composition exhibit a main efficacy to extinguish heat and wet. Most of obesity patients suffer from several diseases resulted from heat and wet from the view of oriental medicine. Therefore, it is thought that the removal of heat and wet can dispel the problems of obesity patients.

The ingredients of the present composition will be described in more detail as follows.

Ephedrae Herba removes ill heat in body by virtue of perspiration and prevents rigor infiltration into body and heat generation. Roasted Ephedrae Herba together with honey strengthens lungs, stops a cough and removes phlegm. The pharmacological effect of ephedrine is similar to that of adrenaline and exhibits slowly for a long period of time. Ephedrine results in significant excitation in central nerve system through the relaxation of smooth muscle of bronchus, excitation of heart, contraction of blood vessel and alleviation of blood pressure. Ephedrae Herba exhibits anti-fatigue effect in skeletal muscle. In addition, the ingredients in Ephedrae Herba inhibit the proliferation of Influenza virus. In the present composition, the amount of Ephedrae Herba ranges from 30 to 60 weight parts. If the amount used is less than 30 weight parts, its efficacy to prevent obesity becomes negligible, while if it exceeds 60 weight parts its efficacy is not improved even when its amount is further increased.

Scutellariae Radix is a root of *Scutellaria baicallensis* belonging to *Labiatae.* The ingredient affects the level of lipid in blood, shows anti-inflammatory effect, anti-allergic effect, antimicrobial effect, antipyretic effect, diuretic effect and the effect of lowering blood pressure. Scutellariae Radix treats conditions resulting from fever toxicity accumulated in body. The ingredient treats rigor and heat generated in bone, extinguishes thirst due to heat. Furthermore, it exhibits efficacy in treating jaundice, dysentery, diarrhea and gastric fever. It mitigates expectoration and promotes the circulation of energy in body. Purulent diseases such as amygdalitis, abscess on breast or back and malignant abscess resulting from fever toxicity can be treated with Scutellariae Radix. In the present composition, the amount of Scutellariae Radix ranges from 30 to 60 weight parts. If the amount is less than 30 weight parts, its efficacy to prevent obesity becomes negligible; but if it exceeds 60 weight parts, its efficacy is not improved even when its amount is further increased.

Typhae Pollen is a pollen of *Typha latifolia* belonging to *Typhaceae.* Since it can purify blood and stanch bleeding, it is useful in bleeding with fever. When there is a sudden nasal bleeding due to inflammation in nose, Typhae Pollen is topically or orally applied. The ingredient is effective in treating symptoms such as vomiting of blood, bloody phlegm, bleeding due to abscess, stomach bleeding and bleeding associated with hemorrhoids. In addition, it is effective in treating acute bleeding of urinary organs and bloody urine of nephrotuberculosis, since it may improve bowel movement and diuresis. The ingredient shows the effects of uterine contraction and bleeding stanching. In the present composition, the amount of Typhae Pollen ranges from 30 to 60 weight parts. If the amount is less than 30 weight parts, its efficacy to prevent obesity becomes negligible; but if it exceeds 60 weight parts, its efficacy is not improved even when its amount is increased further.

Acorus gramineus Rhizome is a subterranean stem of *Acorus gramineus* belonging to *Araceae.* It removes expectoration, so that it promotes circulation of energy and blood in body, and removes wet. The ingredient acts on brain to be effective in amnesia, nonsense and insecurity through removing stagnation of water in body. In addition, it is effective in treating sore condition due to wet. In the present composition, a random amount of Acorus gramineus Rhizome in the range of from 30 to 60 weight parts is added.

Armeniacae Semen is a mature seed of *Prunus armeniaca* belonging to *Rosaceae.* In oriental medicine, it has been mostly used for stopping cough by promoting lung energy and for improving bowel movement. Among ingredients of Armeniacae Semen, the hydrolysates of amygdalin inhibit the activity of the respiratory center to stabilize a respiratory movement, thereby stopping cough. In the present composition, a random amount of Armeniacae Semen in the range of from 30 to 60 weight parts of is added.

Nelumbinus Folium is a leaf of *Nelumbo nucifera* belonging to *Nymphaeaceae.* The ingredient elevates virility to loosen ill blood in body. Therefore, it may be effective in treating vomiting of blood, bloody phlegm, postpartal extravasated blood and pain due to a bruise. In addition, it has been revealed to discharge wet to treat edema. In the present composition, a random amount of Nelumbinus Folium in the range of from 15 to 45 weight parts is added.

Polygalae Radix is a root of *Polygala tenuifolia* belonging to *Polygalaceae.* It is effective in stabilization of spirit, clarification of brain condition, and alleviating gloominess. According to oriental medicine, Shin means to a spirit, and Polygalae Radix has been reported to involve in actions of Shin. It is effective in pectoral effect, excitation on uterus and loosening energy. In the present composition, a random amount of Polygalae Radix in the range of from 15 to 45 weight parts is added. Combinations comprising the ingredients of the present composition known in the art refer to :
Patent CN 1 255 306 A which discloses a process for preparing bio-functional beverages and a series of pharmaceutical compositions which comprise among other constituents Herba Ephedra extract, Radix Scutellaria extract, Herba Typhae extract, being applicable for the treatment of obesity as well as in other different diseases and disorders.
Patent CN 1 043 625 A disclosing a body building and weight reducing preparation for treating obesity (a fat reducing ointment) which comprises Radix Scutellaria and Herba Ephedra among other constituents.
Patent KR 2001 025 156 A which discloses a composition containing a medicinal herb extract which comprises Ephedra Herb and Typhae pollen which inhibits the activity of pancreatic esterase specifically, and decreases the internal absorption of cholesterol. The document of CHO E.J. ET AL (J.KOREAN SOC. FOOD SCI. NUTR. vol. 28, no. 4, 1999, pages 816 - 821) teaches that (-)-ephedrine present in Ephedra herba inhibits pancreatic cholesterol esterase, and thus cholesterol uptake. None of these two documents indicate that this mechanism of action is involved in obesity.

In a process for preparing the present composition, it is preferred that the ingredients described above are mixed as dried material at the ratio described above and 5-15 fold amount of distilled water or alcohol solution are added and extracted. Alternatively, each extract from the ingredients is mixed to obtain a mixture with the ingredient ratio described above. The extract is filtered, concentrated under vacuum and frozen at a temperature below -40°C, followed by freeze-drying under less than 0.2 Torr to obtain powder. Alternatively, the residual concentrated under vacuum is separated using hydrogenated low grade alcohol, concentrated under vacuum to remove residual solvent and subject to freeze-drying as described above, finally yielding powder. The final product is advantageous in terms of administration.

When the present composition is formulated to provide a pharmaceutical composition, it may be administered orally or parentally and may be formulated to provide a general drug form.

The present composition may be provided for oral or parental administration, using a general diluent or a carrier for formulation including a filler, a thickener, a binder, a wetting agent, a disintegrating agent and a surfactant.

The solid formulation for oral administration includes a tablet, a pill, powder, granule and a capsule, which may be prepared by using lignan and lactone compounds and its derivatives together with at least one carrier, for example, starch, calcium carbonate, sucrose, lactose and gelatin. In addition, lubricant such as magnesium stearate or talc as well as a general carrier may be incorporated. The solution formulation for oral administration includes suspension, solution for internal application, emulsion and syrup, which may be prepared by using a carrier such as a wetting agent, a sweetner, aromatic and a preservative as well as a general diluent such as water, liquid paraffin.

The formulation for parenteral administration includes sterilized aqueous solution, non-aqueous solution, suspension, emulsion, freeze-dried preparation and suppositories. For non-aqueous solution and suspension, propylene glycol, polyethylene glycol, plant oil such as olive oil and esters for injection such as ethyloleate may be used. For suppositories, Witepsol, Macrogol, Tween 61, Cacao butter, laurine oil and glycerol gelatin may be used.

The amount of the present composition as an active ingredient in the formulation may be determined depending on absorption rate, inactivation rate and excretion rate of active ingredient in body, age, sex and physical condition of a patient. Generally, the amount ranges from 10 to 200'mg/kg, preferably, 20-100 mg/kg. It may be administered 1-3 times/ day.

In addition, the present invention provides a health food for treating obesity comprising the present composition as an active ingredient.

The health food refers to a food that can exhibit a specific physiological efficacy of interest when ingested, which is prepared by using general ingredients of food and is very advantageous in sustained administration since it shows little or no adverse effects. The health food may be prepared in the form of general food, a capsule, powder and a suspension.

### BRIEF DESCRIPTION OF THE DRAWINGS

Fig. 1 is a flow chart representing the process for preparing the present composition of Example 1.
Fig. 2 is a flow chart representing the process for preparing the present composition of Example 2.
Fig. 3 is a flow chart representing the process for preparing the present composition of Example 3.
Fig. 4 is a flow chart representing the process for preparing the present composition of Example 4.
Fig. 5 is a flow chart representing the process for preparing the present composition of Example 5.
Fig. 6 is a flow chart representing the process for preparing the present composition of Example 6.
Fig. 7 is a flow chart representing the process for preparing the present composition of Example 7.
Fig. 8 is a graph showing the influence of the present composition on body weight of C57BL/6 mouse(Experimental Example 1).
Fig. 9 is a graph showing the influence of the present composition on adipose tissue of epididymis of C57BL/6 mouse(Experimental Example 2).
Fig. 10 is a graph showing the influence of the present composition on postrenal adipose tissue of C57BL/6 mouse(Experimental Example 2).
Fig. 11 is a graph showing the influence of the present composition on neutral lipids of C57BL/6 mouse(Experimental Example 3).
Fig. 12 is a graph showing the influence of the present composition on body weight of ob/ob mouse(Experimental Example 4).

### EXAMPLE 1

10-fold weight of distilled water was added to 135 g of the combined ingredients of herbal medicines containing 45 g of each of Ephedrae Herba, Scutellariae Radix and Typhae Pollen. The extraction was performed at 100°C for 3 hr. The extract was filtered, the filtrate was frozen at -70°C for 24 hr and then subject to freeze-drying at -50°C under 5 mTorr, yielding 27.0 g of powder (extraction yield: 20.0%).

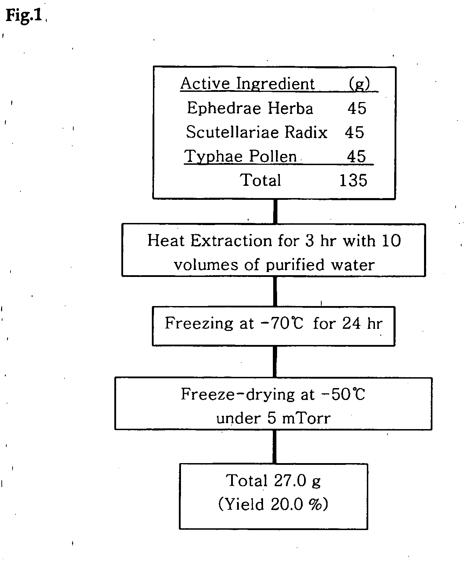

The overall procedure is shown in Fig.1.

### EXAMPLE 2

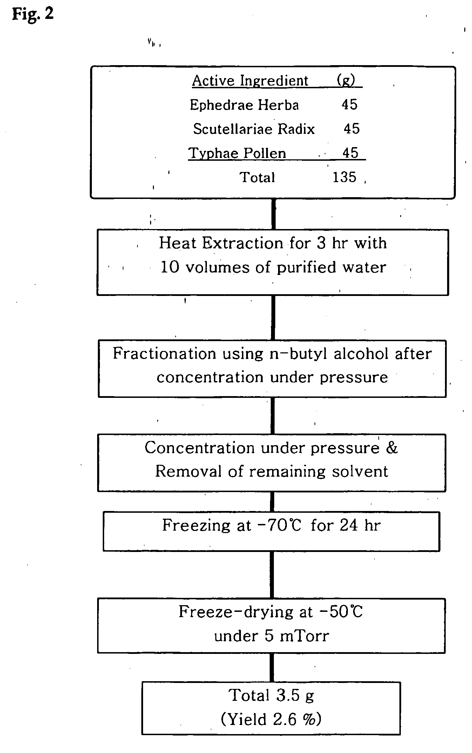

The filtrate produced in Example 1 was concentrated under vacuum to decrease its volume to 5 volumes (v/w) relative to the weight of ingredients of herbal medicines. To the resultant, an equal volume of hydrosaturated normal-butyl alcohol was added, the mixture was applied to separation funnel and agitated, and kept to stand, thereby separating the normal-butyl alcohol layer. After performing refractionation 2-3 times, the layers of normal-butyl alcohol was concentrated under vacuum to completely remove the solvent, and then subject to freeze-drying as Example 1, obtaining 3.5 g of powder (extraction yield: 2.6%). The overall procedure is schematically represented in Fig. 2.

### EXAMPLE 3

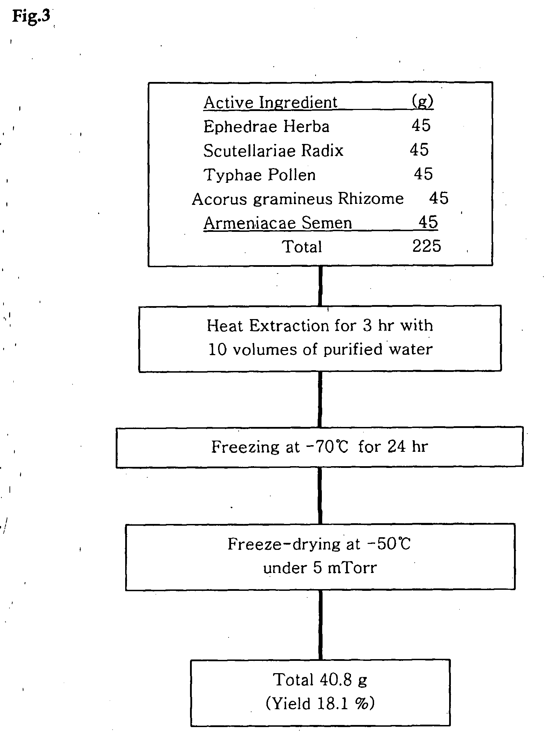

10-fold weight of distilled water was added to 225 g of the combined ingredients of herbal medicines containing 45 g of each of Ephedrae Herba, Scutellariae Radix, Typhae Pollen, Acorus gramineus Rhizome and Armeniacae Semen. The extraction was performed at 100°C for 3 hr. The extract was filtered, the filtrate was frozen at -70°C for 24 hr and then subject to freeze-drying at -50°C under 5 mTorr, yielding 40.8 g of powder (extraction yield:18.1%). The overall procedure is shown in Fig. 3.

### EXAMPLE 4

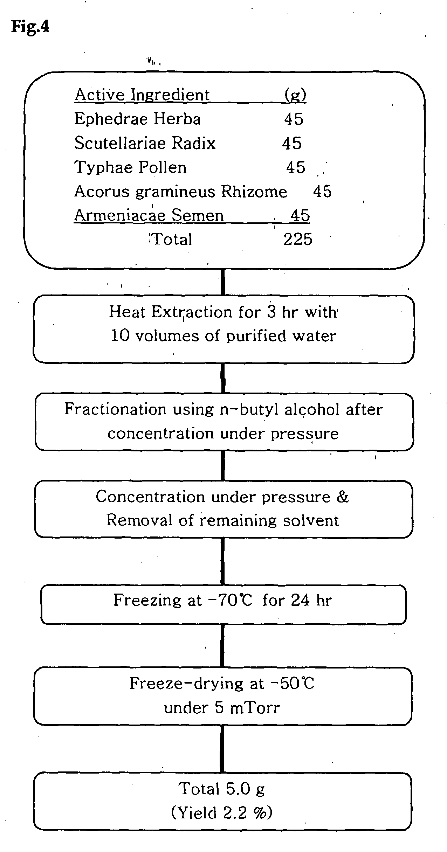

The filtrate produced in Example 3 was concentrated under vacuum to decrease its volume to 5 volumes (v/w) relative to the weight of ingredients of herbal medicines. To the resultant, an equal volume of hydrosaturated normal-butyl alcohol was added, the mixture was applied to separation funnel and agitated, and kept to stand, thereby separating the normal-butyl alcohol layer. After performing refractionation 2-3 times, the layers of normal-butyl alcohol was concentrated under vacuum to completely remove the solvent, and then subject to freeze-drying as Example 3, obtaining 5.0 g of powder (extraction yield: 2.2%). The overall procedure is schematically represented in Fig. 4.

### EXAMPLE 5

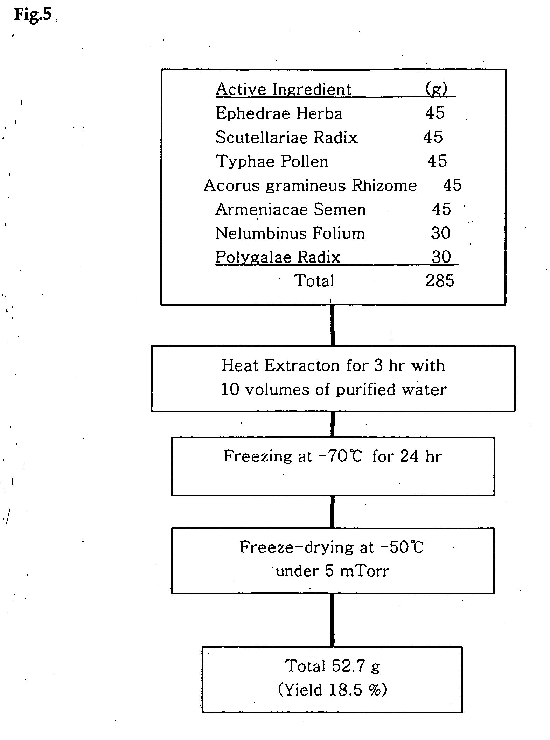

10-fold weight of distilled water was added to 285 g of the combined ingredients of herbal medicines containing 45 g of each of Ephedrae Herba, Scutellariae Radix, Typhae Pollen, Acorus gramineus Rhizome and Armeniacae Semen and 30 g of each of Nelumbinus Folium and Polygalae Radix. The extraction was performed at 100°C for 3 hr. The extract was filtered, the filtrate was frozen at 70°C for 24 hr and then subject to freeze-drying at -50°C under 5 mTorr, yielding 52.7 g of powder (extraction yield: 18.5%). The overall procedure is shown in Fig. 5.

### EXAMPLE 6

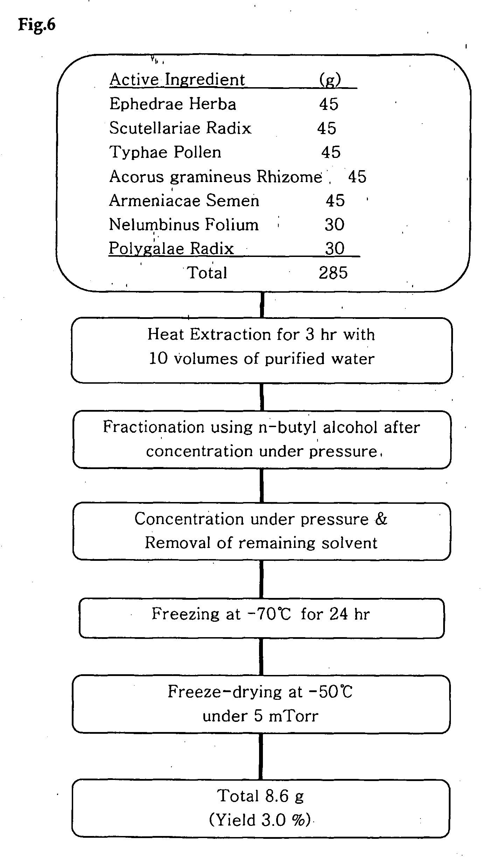

The filtrate produced in Example 5 was concentrated under vacuum to decrease its volume to 5 volumes (v/w) relative to the weight of ingredients of herbal medicines. To the resultant, an equal volume of hydrosaturated normal-butyl alcohol was added, the mixture was applied to separation funnel and agitated, and kept to stand, thereby separating the normal-butyl alcohol layer. After performing refractionation 2-3 times, the layers of normal-butyl alcohol was concentrated under vacuum to completely remove the solvent, and then subject to freeze-drying as Example 5, obtaining 8.6 g of powder (extraction yield: 3.0%). The overall procedure is schematically represented in Fig. 6.

### EXAMPLE 7

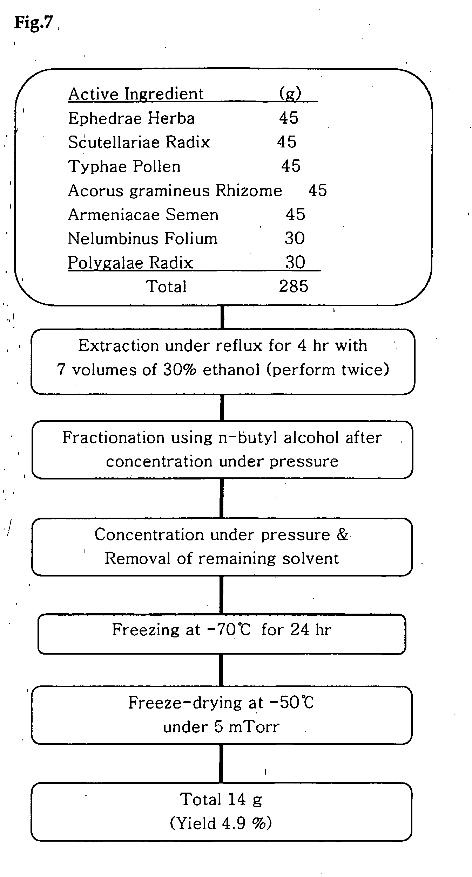

Seven-fold weight of 30% ethanol solution was added to 285 g of the combined ingredients of herbal medicines containing 45 g of each of Ephedrae Herba, Scutellariae Radix, Typhae Pollen, Acorus gramineus Rhizome and Armeniacae Semen and 30 g of each of Nelumbinus Folium and Polygalae Radix. The extraction was performed at 80°C for 4 hr. The extract was filtered, the filtrate was concentrated under vacuum to remove ethanol and the resultant was suspended in distilled water. An equal volume of hydrosaturated normal-butyl alcohol was added, the mixture was applied to separation funnel and agitated, and kept to stand, thereby separating the normal-butyl alcohol layer. After performing refractionation 2-3 times, the layers of normal-butyl alcohol was concentrated under vacuum to completely remove the solvent, and the resultant was frozen at -70°C for 24 hr and then subject to freeze-drying at -50°C under 5 mTorr, yielding 14 g of powder (extraction yield: 4.9%). The overall procedure is schematically represented in Fig. 7.

### EXPERIMENTAL EXAMPLE 1: Efficacy on Weight Loss (Animal Test)

Male C57BL/6 mice aged 4 weeks were used for experiments fed with the fodder indicated in Table 1, which was designed for enabling the mice to ingest about 30% of total calories from fat. The composition of the feed is based on AIN-93G as follows:

Eight experimental groups were divided: control was administered with distilled water and other groups were administered with 500 mg/kg/day, 65 mg/kg/day, 500 mg/kg/day, 60 mg/kg/day, 500 mg/kg/day, 80 mg/kg/day and 87 mg/kg/day of compositions of Examples 1-7, respectively. The administration was orally performed once a day for 8 weeks.

**TABLE 1**

| Composition of Feed | |
|---|---|
| Ingredients | g |
| Casein | 20 |
| Sucrose | 10 |
| Maize starch | 31 |
| Dextrose | 10 |
| Lard(80%) | 13.5 |
| Soy bean | 5.5 |
| Cellulose | 5 |
| Vitamins mix | 1 |
| Minerals mix | 3.5 |
| Cholin bitartarate | 0.2 |
| L-cysteine | 0.3 |
| Butylhydroxyquinoline | 0.0014 |
| Total | 100 |

**TABLE 2**

| Composition of 3 Nutrients in Feed | | | |
|---|---|---|---|
| Nutrients | g | cal | cal % |
| Carbohydrate | 51 | 204 | 47.1284 |
| Protein | 20.54 | 82.16 | 18.98073 |
| Lipid | 16.3 | 146.7 | 33.89087 |
| Total | 87.84 | 432.86 | 100 |

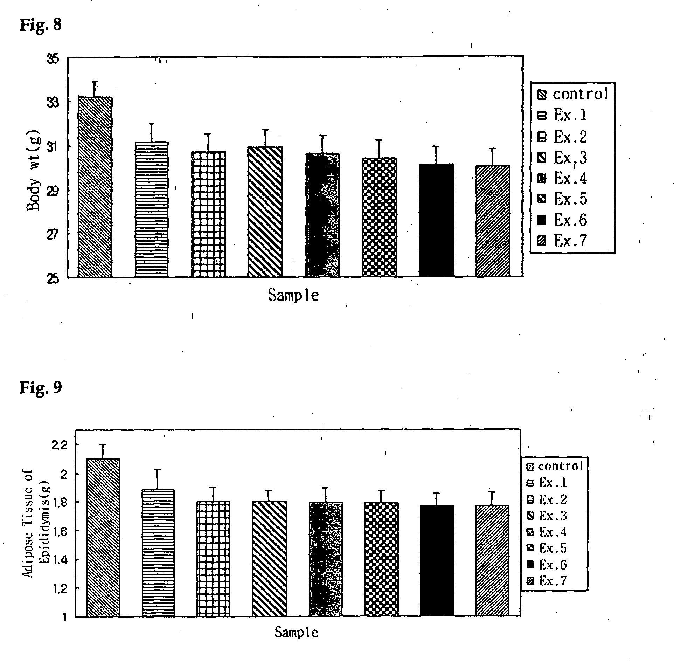

The C57BL/6 mice were weighted after the 8-week long administration. The results are summarized in Table 3 and Fig. 8.

**TABLE 3**

| Cat. | Control | Exa.1 | Exa.2 | Exa.3 | Exa.4 | Exa.5 | Exa.6 | Exa.7 |
|---|---|---|---|---|---|---|---|---|
| Weight | 33.19 | 31.18 | 30.72 | 30.94 | 30.61 | 30.42 | 30.12 | 30.03 |
| (g) | ±0.70 | ±0.85 | ±0.81 | ±0.80 | ±0.84 | ±0.80 | ±0.82 | ±0.78 |
| Mean ±standard error | | | | | | | | |

In view of body weight after 8-week administration, the groups administered with the present compositions of Examples 1-7 exhibited the decreased pattern by 2.01 g(6.1%), 2.47 g(7.4%), 2.25 g(6.8%), 2.58 g(7.8%), 2.77 g(8.3%), 3.07 g(9.2%) and 3.16 g(9.5%).

### EXPERIMENTAL EXAMPLE 2: Influence on Adipose Tissues in Abdominal Cavity

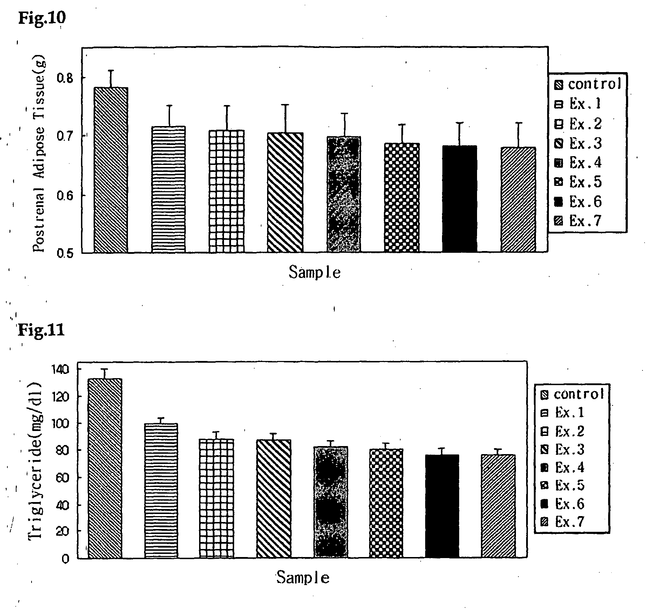

Blood samples were collected from the mice of Experimental example 1 and autopsy was then carried out by separating adipose tissues distributed around the epididymis and kidney in abdominal cavity. The separated tissues were weighted and frozen on dry ice, followed by storing at -70°C. The results are represented in and Figs. 9 and 10.

**TABLE 4**

| Cat. | Control | Exa.1 | Exa.2 | Exa.3 | Exa.4 | Exa.5 | Exa.6 | Exa.7 |
|---|---|---|---|---|---|---|---|---|
| Adipose tissues of epididymis (g) | 2.102 ±0.097 | 1.888 ±0.134 | 1.804 ±0.094 | 1.802 ±0.081 | 1.794 ±0.096 | 1.785 ±0.091 | 1.769 ±0.085 | 1.765 ±0.092 |
| Postrenal adipose tissues (g) | 0.782 ±0.029 | 0.715 ±0.038 | 0.709 ±0.042 | 0.703 ±0.049 | 0.698 ±0.039 | 0.687 ±0.032 | 0.681 ±0.039 | 0.679 ±0.041 |
| Mean ±standard error | | | | | | | | |

In view of adipose tissues of epididymis after 8-week administration, the groups administered with the present compositions of Examples 1-7 exhibited the decreased pattern relative to control by 0.214 g(10.2%), 0.298 g(14.2%), 0.3 g(14.3%), 0.308 g(14.7%), 0.317 g(15.1%), 0.333 g(15.8%) and 0.337 g(16.0%).

In view of postrenal adipose tissues after 8-week administration, the groups administered with the present compositions of Examples 1-7 exhibited the decreased pattern relative to control by 0.067 g(8.6%), 0.073 g(9.3%), 0.079 g(10.1%), 0.084 g(10.7%), 0.095 g(12.1%), 0.101 g(12.9%) and 0.103 g(13.2%).

### EXPERIMENTAL EXAMPLE 3: Change of Blood Lipids

The mice of Experimental example 1 were asphyxiated over 16 hr and blood samples were collected from their hearts. The concentration of neutral lipids was measured using serum. The analysis was carried out by using a kit (Asan Pharmaceuticals, Inc.) using enzyme reaction. The results are summarized in Table 5 and Fig.11.

**TABLE 5**

| Cat. | Control | Exa.1 | Exa.2 | Exa.3 | Exa.4 | Exa.5 | Exa.6 | Exa.7 |
|---|---|---|---|---|---|---|---|---|
| Triglyceride (mg/ dL) | 133.09 ±6.56 | 99.33 ±4.23 | 88.14 ±4.91 | 87.22 ±5.16 | 82.40 ±4.14 | 79.91 ±4.45 | 75.47 ±5.05 | 75.41 ±4.92 |
| Mean ±standard error | | | | | | | | |

In view of neutral lipids (triglyceride) in serum after 8-week administration, the groups administered with the present compositions of Examples 1-7 exhibit the decreased pattern relative to control by 33.74 mg/dℓ(25.4%), 44.93 mg/dℓ(33.8%), 45.85 mg/dℓ(34.5%), 50.67 mg/dℓ(38.1%), 53.16 mg/dℓ(39.9%), 57.6 mg/dℓ(43.3%) and 57.66 mg/dℓ(43.3%).

### EXPERIMENTAL EXAMPLE 4: Change of Body Weight

Male ob/ob mice aged 6 weeks were provided by Harlan Lab and kept to be freely accessible to feed and water to adapt to our Lab for 1 week. Afterwards, they were weighted (0 day) and distributed to show the identical mean weight. The animals were classified to 9 groups and then the control was administered with a carrier, the positive control with reductil, and experimental groups with the compositions of Examples 1-7, respectively. The dosage is the same as that of Experimental example 1. The administration was orally performed once a day for 4 weeks.

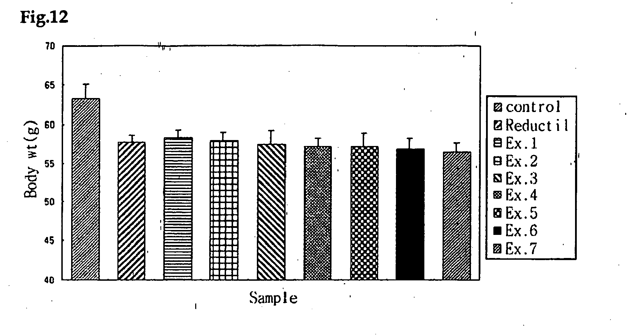

During 4-week administration, the change of body weight was exmained. The efficacy was evaluated with comparing the prevention effect on body weight increase to that of control. The results are summarized in Table 6 and Fig. 12.

**TABLE 6**

| Cat. | Control | Reductil | Exa.1 | Exa.2 | Exa.3 | Exa.4 | Exa.5 | Exa.6 | Exa.7 |
|---|---|---|---|---|---|---|---|---|---|
| Body weight (g) | 63.22 ±1.87 | 57.69 ±0.92 | 58.21 ±1.12 | 57.91 ±1.07 | 57.31 ±1.85 | 57.1 ±1.01 | 57.13 ±1.73 | 56.82 ±1.27 | 56.41 ±1.15 |
| Mean ±standard error | | | | | | | | | |

Following 4-week administration, the body weight of control showed 63.22 g while that of positive control administered with reductil showed 57.69 g, which represents the decrease by 5.53 g(8.7%). The groups administered with the present compositions of Examples 1-7 exhibited the body weight of 58.21 g, 57.91 g, 57.31 g, 57.1 g, 57.13 g and 56.41 g, respectively. These results represent the decreased pattern by 5.01 g(7.9%), 5.31 g(8.4%), 5.91 g(9.3%), 6.12 g(9.7%), 6.09 g(9.7%), 6.4 g(10.1%) and 6.81 g(10.8%).

### FORMULATION EXAMPLE 1: Powder and Capsules

14.8 mg of lactose, 3 mg of crystalline cellulose, 0.2 mg of magnesium stearate and 200 mg of extract of Examples 1, 3 or 5, or 100 mg of fraction of Examples 2, 4, 6 or 7 were mixed. Gelatin capsules of No. 5 were charged with the mixture using suitable apparatus.

The composition of the powder and capsule is as follows:

| | |
|---|---|
| Active ingredient | 200 mg (Examples 1, 3 and 5) |
| | 100 mg (Examples 2, 4, 6 and 7) |
| Lactose | 14.8 mg |
| Crystalline cellulose | 3 mg |
| Magnesium stearate | 0.2 mg |

### FORMULATION EXAMPLE 2: Solution for Injection

180 mg of mannitol, 26 mg of Na2HPO₄ . 12H₂O, 2974 mg of distilled water and 100 mg of extract of Examples 1, 3 or 5, or 50 mg of fraction of Examples 2, 4, 6 or 7 were mixed. The mixture was poured into bottle and sterilized at 20°C for 30 min.

| | |
|---|---|
| Active ingredient | 100 mg (Examples 1, 3 and 5) |
| | 50 mg (Examples 2, 4, 6 and 7) |
| Mannitol | 180 mg |
| Na2HPO₄ · 12H₂O | 26 mg |
| Distilled water | 2974 mg |

### FORMULATION EXAMPLE 3: Health food

For 1-day administration, powdered vitamin E, ferric lactate, zinc oxide, nicotinic acid amide, vitamin A, vitamin B1, vitamin B2 and 400 mg of extract of Examples 1, 3 or 5, or 200 mg of fraction of Examples 2, 4, 6 or 7 were mixed.

The composition of the health food is as follows (1-day dose for human):

| | |
|---|---|
| Active ingredient | 400 mg (Examples 1, 3 and 5) |
| | 200 mg (Examples 2, 4, 6 and 7) |
| Vitamin C | 100 mg |
| Powdered Vit. E | 120 mg |
| Ferric lactate | 2 mg |
| Zinc oxide | 2 mg |
| Nicotinic acid amide | 20 mg |
| Vitamin A | 5 mg |
| Vitamin B1 | 2 mg |
| Vitamin B2 | 2mg |
| Maize starch | 200 mg |
| Magnesium stearate | 20 mg |

### Industrial Applicability

As described above, the present composition can reduce body weight, adipose tissues and neutral lipids, thus showing an excellent therapeutic effect for preventing obesity. Accordingly, the present composition is very useful as health food and a therapeutic agent for use in treating diseases associated with obesity as well as reducing body weight.

## Claims

1. A composition for use in preventing and/or treating obesity consisting of water extracts or aqueous alcoholic solution extracts of, as dry weight,
a) 30-60 weight parts of Ephedrae Herba, Scutellariae Radix and Typhae Pollen, respectively, or
b) 30-60 weight parts of Ephedrae Herba, Scutellariae Radix and Typhae Pollen, respectively, and at least one selected from the group consisting of water extracts or aqueous alcoholic solution extracts of, as dry weight, 30-60 weight parts of Acorus gramineus Rhizome, water extracts or aqueous alcoholic solution extracts of, as dry weight, 30-60 weight parts of Armeniacae Semen, water extracts or aqueous alcoholic solution extracts of, as dry weight, 15-45 weight parts of Nelumbinus Folium and water extracts or aqueous alcoholic solution extracts of, as dry weight, 15-45 weight parts of Polygalae Radix.

2. An agent for treating obesity comprising the composition of claim 1 as an active ingredient.

3. A health food comprising the composition of claim 1.

4. Use of a composition according to claim 1 or of an agent according to claim 2 for the manufacture of a medicament for preventing and/or treating obesity.

5. A process for preparing a composition for preventing and/or treating obesity, wherein said process comprises steps of:
extracting a mixture of 30-60 weight parts of each of Ephedrae Herba, Scutellariae Radix and Typhae Pollen as dry weight in hot water or an aqueous alcoholic solution;
concentrating said extract under vacuum and separating said extract by using an equal amount of a lower alcohol or a nonpolar solvent, followed by concentrating the resultant under vacuum;
azetotropically concentrating the resulting concentrate by virtue using water; and
freeze-drying the resultant to obtain a powdered extract.

6. The process according to claim 5, wherein said mixture further comprises at least one selected from the group consisting of 30-60 weight parts of Acorus gramineus Rhizome, 30-60 weight parts of Armeniacae Semen, 15-45 weight parts of Nelumbinus Follum and 15-45 weight parts of Polygalae Radix.

## Patentansprüche

1. Zusammensetzung zur Verwendung bei der Prävention und/oder Behandlung von Obesität, bestehend aus wässrigen Extrakten oder aus Extrakten mit einer wässrig-alkoholischen Lösung von, in Trockengewicht,
a) jeweils 30-60 Gewichtsteilen Ephedrae Herba, Scutellariae Radix und Typhae Pollen oder
b) jeweils 30-60 Gewichtsteilen Ephedrae Herba, Scutellariae Radix und Typhae Pollen und wenigstens einem, ausgewählt aus der Gruppe, bestehend aus wässrigen Extrakten oder Extrakten mit einer wässrig-alkoholischen Lösung von, in Trockengewicht, 30-60 Gewichtsteilen Acorus gramineus-Rhizomen, wässrigen Extrakten oder Extrakten mit einer wässrig-alkoholischen Lösung von, in Trockengewicht, 30-60 Gewichtsteilen Armeniacae Semen, wässrigen Extrakten oder Extrakten mit einer wässrig-alkoholischen Lösung von, in Trockengewicht, 15-45 Gewichtsteilen Nelumbinus Folium und wässrigen Extrakten oder Extrakten mit einer wässrig-alkoholischen Lösung von, in Trockengewicht, 15-45 Gewichtsteilen Polygalae Radix.

2. Mittel zur Behandlung von Obesität, welche die Zusammensetzung nach Anspruch 1 als aktiven Inhaltsstoff umfaßt.

3. Gesundheitskost, welche die Zusammensetzung nach Anspruch 1 umfaßt.

4. Verwendung einer Zusammensetzung nach Anspruch 1 oder eines Mittels nach Anspruch 2 für die Herstellung eines Medikaments zur Prävention und/oder für die Behandlung von Obesität.

5. Verfahren zum Herstellen einer Zusammensetzung zur Prävention und/oder für die Behandlung von Obesität, wobei das Verfahren die folgenden Stufen umfaßt:
Extrahieren eines Gemischs von, in Trockengewicht, 30-60 Gewichtsteilen von jedem von Ephedrae Herba, Scutellariae Radix und Typhae Pollen in heißem Wasser oder einer wässrig-alkoholischen Lösung,
Konzentrieren des Extrakts unter Vakuum und Auftrennen des Extrakts unter Verwendung einer gleichen Menge eines niederen Alkohols oder eines nicht polaren Lösungsmittels, gefolgt von Konzentrieren des resultierenden Produkts unter Vakuum,
azeotropes Konzentrieren des resultierenden Konzentrats durch die Verwendung von Wasser und
Gefriertrocknen des resultierenden Produkts unter Erhalt eines pulverförmigen Extrakts.

6. Verfahren nach Anspruch 5, wobei das Gemisch weiterhin wenigstens eines umfaßt, ausgewählt aus der Gruppe, bestehend aus 30-60 Gewichtsteilen Acorus gramineus-Rhizomen, 30-60 Gewichtsteilen Armeniacae Semen, 15-45 Gewichtsteilen Nelumbinus Folium und 15-45 Gewichtsteilen Polygalae Radix.

## Revendications

1. Composition à utiliser dans la prévention et/ou le traitement de l'obésité constituée par des extraits aqueux ou des extraits en solution alcoolique aqueuse, en poids sec, de :
a) 30 à 60 parties en poids de Ephedrae Herba, Scutellariae Radix et Typhae Pollen, respectivement, ou
b) 30 à 60 parties en poids de Ephedra Herba, Scutellariae Radix et Typhae Pollen, respectivement, et d'au moins l'un choisi dans le groupe constitué par des extraits aqueux ou des extraits en solution alcoolique aqueuse, en poids sec, de 30 à 60 parties en poids de Acorus gramineus Rhizome, des extraits aqueux ou des extraits en solution alcoolique aqueuse, en poids sec, de 30 à 60 parties en poids de Armeniacae Semen, des extraits aqueux ou des extraits en solution alcoolique aqueuse, en poids sec, de 15 à 45 parties en poids de Nelumbinus Folium et des extraits aqueux ou des extraits en solution alcoolique aqueuse, en poids sec, de 15 à 45 parties en poids de Polygalae Radix.

2. Agent destiné au traitement de l'obésité comprenant la composition selon la revendication 1, comme ingrédient actif.

3. Aliment naturel comprenant la composition selon la revendication 1.

4. Utilisation d'une composition selon la revendication 1 ou d'un agent selon la revendication 2, pour la fabrication d'un médicament destiné à la prévention et/ou au traitement de l'obésité.

5. Procédé pour la préparation d'une composition destinée à la prévention et/ou au traitement de l'obésité, dans lequel ledit procédé comprend les étapes consistant à :
- extraire un mélange de 30 à 60 parties en poids de chacun de Ephedrae Herba, Scutellariae Radix et Typhae Pollen, en poids sec, dans de l'eau chaude ou dans une solution alcoolique aqueuse ;
- concentrer ledit extrait sous vide et séparer ledit extrait en utilisant une quantité égale d'un alcool inférieur ou d'un solvant apolaire, étape suivie par la concentration du produit résultant sous vide ;
- concentrer de manière azéotropique le concentré résultant en utilisant de l'eau ; et
- lyophiliser le produit résultant afin d'obtenir un extrait en poudre.

6. Procédé selon la revendication 5, dans lequel ledit mélange comprend en outre au moins l'un choisi dans le groupe constitué par 30 à 60 parties en poids de Acorus gramineus Rhizome, 30 à 60 parties en poids de Armeniacae Semen, 15 à 45 parties en poids de Nelumbinus Folium et 15 à 45 parties en poids de Polygalae Radix.
